# EUROPÄISCHE PATENTSCHRIFT

(11) **EP 3 905 992 B1**
(45) Veröffentlichungstag und Bekanntmachung des Hinweises auf die Patenterteilung: **19.02.2025**
(21) Anmeldenummer: 21702593.1
(22) Anmeldetag: 15.01.2021
(51) Int. Cl.: A61F 2/44, A61F 2/30

(54) **ZWISCHENWIRBELIMPLANTAT**
INTERVERTEBRAL IMPLANT
IMPLANT INTERVERTÉBRAL

(30) Priorität: 21.01.2020 DE 102020000319
(43) Veröffentlichungstag der Anmeldung: 10.11.2021
(73) Patentinhaber: Joimax GmbH, 76227 Karlsruhe (DE)
(72) Erfinder: RIES, Wolfgang, 76351 Linkenheim Baden-Württemberg (DE); BARTHOLD, Clemens, 76133 Karlsruhe (DE); SCHEINOST, Tobias, 76131 Karlsruhe (DE)
(74) Vertreter: Lichti - Patentanwälte Partnerschaft mbB
(86) Internationale Anmeldenummer: PCT/EP2021/050788
(87) Internationale Veröffentlichungsnummer: WO 2021/148316

(56) Entgegenhaltungen:
- US-A1- 2017 333 203
- US-A1- 2019 269 521

## Beschreibung

Die Erfindung betrifft ein Zwischenwirbelimplantat mit mindestens zwei oberen und zwei unteren Kontaktkörpern mit Kontaktflächen, mit einem Aktuator mit einem eine Erstreckungsachse aufweisenden Gewindekörper, der mit hintereinander angeordnete gegenläufigen Gewinden versehen ist, mit auf dem Gewindekörper axial beweglich aufsitzenden, durch Verdrehung desselben entlang diesen verfahrbaren Keilen mit wenigstens an Gegenflächen zumindest eines Teils der Kontaktkörper angreifenden, unter einem endlichen Winkel kleiner 90°aufeinander zu verlaufenden Rampen mindestens eines Rampenkörpers eines Keils.

Die US 2019/0269521 A1 zeigt ein gattungsgemäßes Zwischenwirbelimplantat, bei dem einzelne Kontaktkörper des Zwischenimplantats seitlich auseinanderverfahren und in vertikaler Richtung auseinanderbewegt werden können. Das seitliche Auseinanderverfahren geschieht durch einen Aktuator mit einer Gewindestange mit gegenläufigen Gewinden über auf diesen mit Rampen versehenen axial verschiebbaren Gleitkörpern, die an entsprechenden Gegenflächen der Kontaktkörper angreifen.

Das vertikale Auseinanderfahren der Kontaktkörper erfolgt indirekt über zwischen den Keilen und den Tragkörpern angeordneten Zwischenkörpern, die in schräg verlaufenden Nuten der unteren Tragkörper verschiebbar sind.

Eine derartige Ausgestaltung ist aufgrund der als zusätzlicher bewegbarer Teile vorgesehenen Zwischenkörpern zusätzlich zu den Keilen und den Tragkörpern zum einen aufwendig, zum anderen störanfällig, da hierdurch Verkantungen und Verklemmungen entstehen können.

Der Erfindung liegt daher die Aufgabe zugrunde, ein Zwischenwirbelimplantat zu schaffen, das unter Vermeidung der vorgenannten Nachteile einfach aufgebaut ist und eine zuverlässige Funktion sicherstellt unter Ermöglichung einer zeitlich versetzten Bewegung der Tragkörper in verschiedene Richtungen.

Erfindungsgemäß wird die genannte Aufgabe mit einem gattungsgemäßen Zwischenwirbelimplantat gelöst, welches dadurch gekennzeichnet ist, dass die Keile als Doppelkeile mit in Richtung der Erstreckungsachse zwei hintereinander angeordneten Rampenkörper ausgebildet sind und dass die Rampen des einen Rampenkörpers zu den Rampen des anderen Rampenkörpers unterschiedliche Ausrichtung aufweisen und dass die Rampen des ersten Rampenkörpers seitlich direkt an den Kontaktkörpern angreifen, während die Rampen des zweiten Rampenkörpers in vertikaler Richtung direkt an den Kontaktkörpern angreifen.

Die Erfindung sieht also zwei axial hintereinander angeordnete Rampenkörper mit unterschiedlicher Ausrichtung der Rampen an unterschiedlichen Rampenkörpern am gleichen Doppelkeil vor, wobei der erste Rampenkörper zum lateralen oder seitlichen Auseinanderbewegen der Tragkörper, der zweite Rampenkörper zum Auseinanderbewegen der Tragkörper in vertikaler oder cephalocaudaler Richtung dient.

Dabei ist insbesondere vorgesehen, dass die Rampen unterschiedlicher Ausrichtung an unterschiedlichen Rampenkörpern eines Doppelkeils angeordnet sind.

Eine äußerst bevorzugte Ausgestaltung der Erfindung sieht vor, dass die Rampen von ersten Rampenkörpern vertikal mit horizontaler Flächennormale ausgerichtet sind und die Flächennormalen der Rampen von zweiten Rampenkörpern in hierzu unterschiedlicher Ausrichtung einen endlichen Winkel ungleich 90° zur Vertikalen einschließen. Dabei steht eine Weiterbildung vor, dass mit Rampen unterschiedlicher Ausrichtung zusammenwirkende Gegenflächen der Kontaktkörper mit einem gegenüber dem Abstand der Rampenkörper unterschiedlicher Ausrichtung unterschiedlichen Abstand in Erstreckungsrichtung der Achse des Gewindekörpers des Aktuators aufweisen, wobei insbesondere vorgesehen ist, dass der Abstand von Rampen unterschiedlicher Ausrichtung relativ zum Abstand der Gegenflächen an den Kontaktkörpern derart ist, dass bei einer Drehung des Gewindekörpers die Kontaktkörper zumindest lateral auseinanderbewegt und erst anschließend relativ zueinander angehoben werden.

Dies kann in konkreter Ausgestaltung dadurch erreicht werden, dass der Abstand der Rampen relativ zum Abstand der Gegenflächen derart ist, dass die Kontaktkörper zuerst durch die Rampen des ersten Rampenkörpers seitlich an den Gegenflächen der Kontaktkörper angreifen, um diese lateral auseinander zu bewegen und erst bei weiterer Verdrehung die Rampen des zweiten Rampenkörpers an den Gegenflächen der Kontaktkörper angreifen, um diese anzuheben. Dadurch wird ein zeitlich versetztes Bewegen des Tragkörpers konkret realisiert.

Auch kann erfindungsgemäß vorgesehen sein, dass der Abstand der Rampen unterschiedlicher Ausrichtung eines Doppelkeils mit zwei Rampenteilen geringer ist als der Abstand der zugehörigen Gegenflächen.

Weitere bevorzugte Ausgestaltungen der Erfindung sehen vor, dass der Aktuator eine fest mit dem Gewindekörper verbundene radiale Scheibe oder ein radiales Rad aufweist, das in radial zur Achse des Gewindekörpers gerichtete Schlitze der Kontaktkörper zur achssenkrechten Führung derselben eingreift und/oder durch zumindest in den oberen Kontaktkörpern gleitend eingreifende Führungsstäbe zur Führung der Kontaktkörper relativ zueinander.

Die äußere Kontaktfläche der oberen Kontaktkörper und die untere Außenfläche der unteren Kontaktkörper verlaufen in der Regel nicht parallel zueinander; es ist vielmehr vorzugsweise vorgesehen, dass die äußere Kontaktfläche von oberen Kontaktkörpern zu der äußeren Kontaktfläche der unteren Kontaktkörper einen Winkel zwischen 5° und 15°, vorzugsweise zwischen 9° und 11° einschließen, um so eine bessere Anpassung an die natürliche Lordose zur Lendenwirbelsäule zu erreichen. Dabei beginnt der proximale Bereich der Kontaktflächen jedes einzelnen Kontaktkörpers zunächst in Relation zur horizontalen Mittelebene gesehen mit der Hälfte dieses Winkels linear ansteigenden Flächen und geht dann im distalen Bereich in eine entgegengesetzte Krümmung über, um eine Rundung zu bilden, die auch das Einführen in den Zwischenwirbelraum erleichtert.

In bevorzugter Ausgestaltung ist vorgesehen, dass die Rampenkörper an einem Doppelkeil einstückig mit diesem ausgebildet sind.

In einer äußerst bevorzugten Ausgestaltung ist vorgesehen, dass übereinanderliegende und/oder nebeneinanderliegende Kontaktkörper über Linearführungen beweglich zueinander verbunden sind, wobei insbesondere zumindest eine Linearführung eine Nut-Feder-Führung, vorzugsweise eine Schwalbenschwanzführung ist. Durch diese Linearführungen werden bei Scherbelastung auftretende Kräfte durch die Kontaktkörper geleitet und beanspruchen innere (Funktions-) Strukturen, wie den Verschiebemechanismus der Doppelschraube, nicht oder zumindest weniger.

Weitere Vorteile und Merkmale der Erfindung ergeben sich aus den Ansprüchen und aus der nachfolgenden Beschreibung, in der ein bevorzugtes Ausführungsbeispiel der Erfindung unter Bezugnahme auf die Zeichnung erläutert ist. Dabei zeigt:
- Fig. 1: Eine Explosionsdarstellung eines ersten erfindungsgemäßen Zwischenwirbelimplantats;
- Fig. 2: eine perspektivische Darstellung des Antriebsmechanismus des erfindungsgemäßen Implantats der Fig. 1 im komprimierten Zustand;
- Fig. 2a: eine Seitenansicht des Antriebsmechanismus der Fig. 2;
- Fig. 3: eine perspektivische Darstellung des Antriebsmechanismus der Ausführungsform der Fig. 1 im expandierten Zustand;
- Fig. 3a: eine Seitenansicht des Antriebsmechanismus entsprechend der Fig. 3;
- Fig. 4: eine perspektivische Darstellung eines Doppelkeils des erfindungsgemäßen Implantats der Fig. 1;
- Fig. 4a: eine Draufsicht auf den Doppelkeil der Fig. 4;
- Fig. 4b: eine Seitenansicht des Doppelkeils der Fig. 4;
- Fig. 5: eine Darstellung des vollständigen Implantats im komprimierten Zustand;
- Fig. 5a: eine Darstellung des Implantats im komprimierten Zustand mit entferntem oberen vorderen Kontaktkörper;
- Fig. 6: eine Darstellung des Implantats gemäß einem ersten Schritt lediglich lateral (seitlich) expandiertem Zustand;
- Fig. 6a: eine Darstellung des Implantats gemäß einem ersten Schritt lediglich lateral (seitlich) expandiertem Zustand gemäß Fig. 6 mit entferntem oberen vorderen Kontaktkörper;
- Fig. 7: das erfindungsgemäße Implantat im nach dem zweiten Expansionsschritt lateral und vertikal expandiertem Zustand;
- Fig. 7a: das erfindungsgemäße Implantat im nach dem zweiten Expansionsschritt lateral und vertikal expandiertem Zustand gemäß Fig. 7 bei entferntem oberen vorderen Tragkörper;
- Fig. 8: eine Explosionsdarstellung einer anderen Ausführungsform des erfindungsgemäßen Zwischenwirbelimplantats;
- Fig. 9: eine perspektivische Darstellung eines anderen Doppelkeils der Ausgestaltung der Fig. 8;
- Fig. 10: eine perspektivische Darstellung des Implantats der Fig. 8 im komprimierten Zustand;
- Fig. 10a: eine Seitenansicht des Implantats der Fig. 8, 10 im komprimierten Zustand;
- Fig. 11: eine Explosionsdarstellung eines Implantats der Fig. 8 im vollständig expandierten Zustand;
- Fig. 11a: eine Seitenansicht des Implantats der Fig. 8 im vollständig expandierten Zustand; und
- Fig. 12: eine abgewandelte Ausführungsform des erfindungsgemäßen Zwischenwirbelimplantats mit Linearführungen in komprimiertem Zustand;
- Fig. 13: die Ausführungsform der Fig. 12 in seitlich oder horizontal expandiertem Zustand;
- Fig. 14: eine Seitenansicht des erfindungsgemäßen Zwischenwirbelimplantats der Fig. 12 im Bereich der - von der Angriffsseite aus gesehen - rechten oberen und unteren Kontaktkörper;
- Fig. 15: eine perspektivische Sicht auf die Innenseite des - von der Angriffsseite aus gesehen - linken oberen Kontaktkörpers; und
- Fig. 16: eine Sicht von unten auf nebeneinanderliegende untere Kontaktkörper.

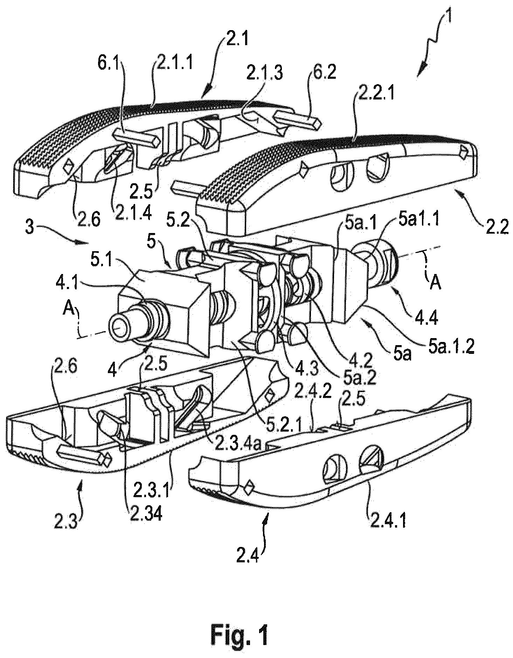

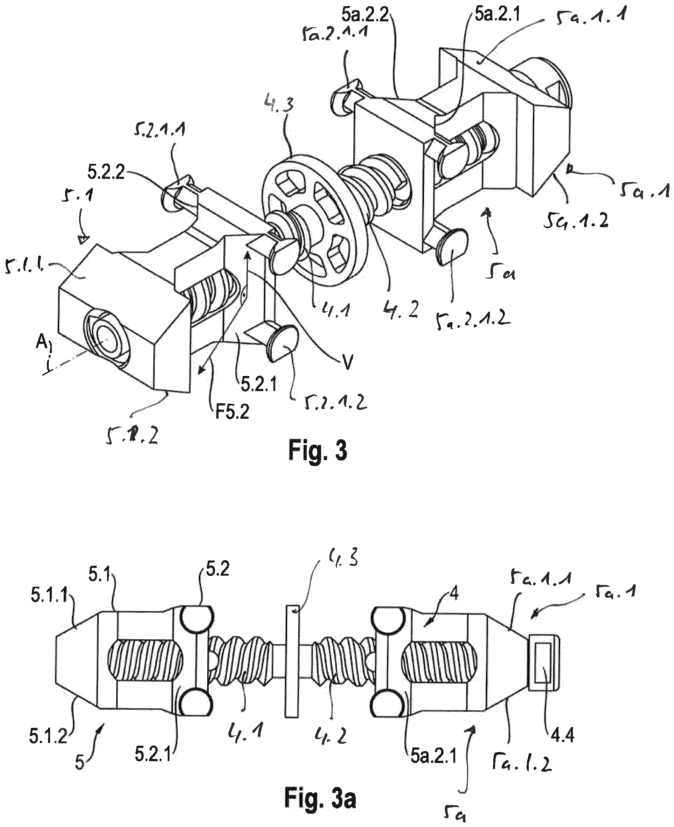

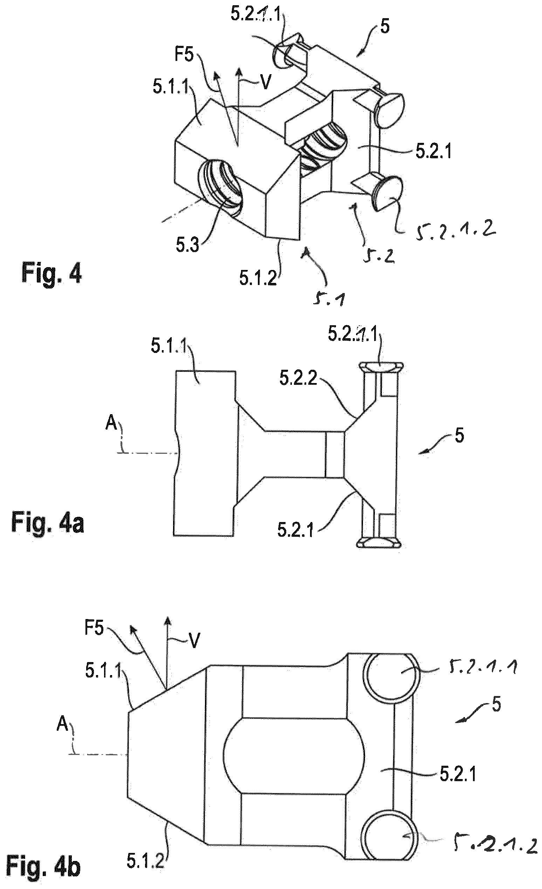

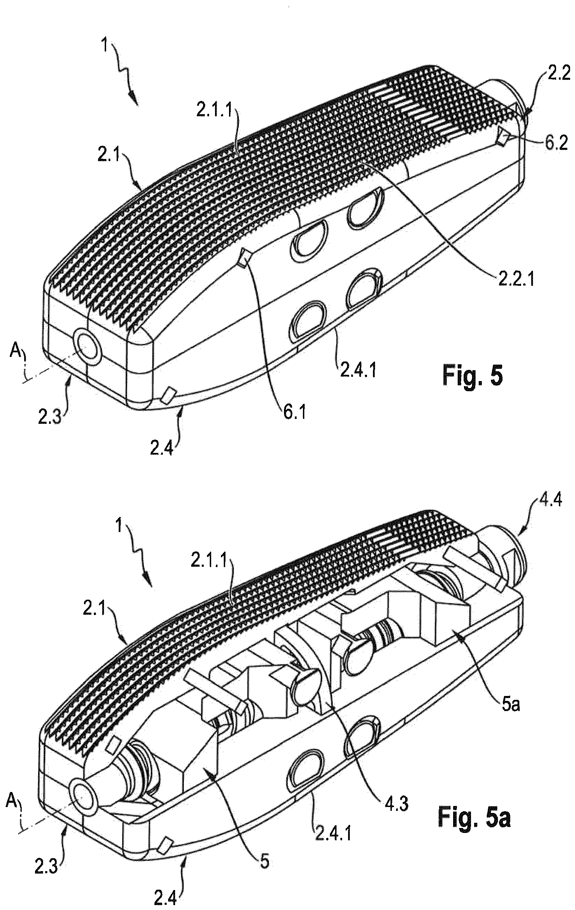

Eine erste Ausführungsform des erfindungsgemäßen Zwischenwirbelimplantats ist in den Fig. 1 bis Fig. 7a dargestellt. Das Implantat 1 weist zwei obere Kontaktkörper 2.1 und 2.2 (2.2 in den Fig. 5a, 6a, 7a entfernt) und diesem gegenüberliegend zwei untere Kontaktkörper 2.3, 2.4 auf. Mittig zwischen diesem ist zentral ein Aktuator oder Antriebsmechanismus 3 angeordnet (insbesondere auch Fig. 3, 3a).

Die Kontaktkörper 2.1-2.4 weisen nach oben bzw. nach unten gerichtete Kontaktflächen auf, die in ihrem Mittelbereich, beispielsweise 2.1.1, 2.2.1, 2.3.1, 2.4.1 im Wesentlichen horizontal gerichtet sind bzw. einen horizontalen Bereich bilden bzw. definieren, während die Kontaktflächen in Längsrichtung an ihren Enden abfallen bzw. gebogen sind und dabei einander gegenüberliegende obere und untere Kontaktflächen aufeinander zu laufen.

Der Antriebsmechanismus 3 weist einen zentralen Gewindekörper 4 mit einer Achse A auf, die auch die Längsrichtung des Implantats bestimmt. Der Gewindekörper 4 hat zwei hintereinander angeordnete Gewinde 4.1, 4.2 mit gegenläufiger Ausrichtung. Das Gewinde 4.1 ist das distale Gewinde, das Gewinde 4.2 das proximale Gewinde. Der Gewindekörper 4 ist am proximalen Ende mit einer Angriffskontur 4.4 zum Angreifen eines Werkzeugs (nicht dargestellt) ausgebildet, mit dem der Gewindekörper 4 verdreht werden kann. Mittig auf dem Gewindekörper 4 ist drehfest mit diesem verbunden ein Führungsrad 4.3 angeordnet, das in radial gerichtete Querschlitze 2.5 der Kontaktkörper 2.1-2.4 eingreift und derart die relative Axialposition der Kontaktkörper 2.1-2.4 und des Gewindekörpers 4 zueinander bestimmt und festlegt - unabhängig von den lateralen und vertikalen Bewegungen der Kontaktkörper 2.1-2.4 relativ zueinander.

Auf dem Gewindekörper 4 sitzen mit an die Gewinde 4.1, 4.2 angepassten Innengewinden 5.3 versehene Doppelkeile 5, 5a mit zwei Rampenteilen. Diese sind spiegelbildlich ausgebildet und spiegelbildlich beiderseits des Rads angeordnet.

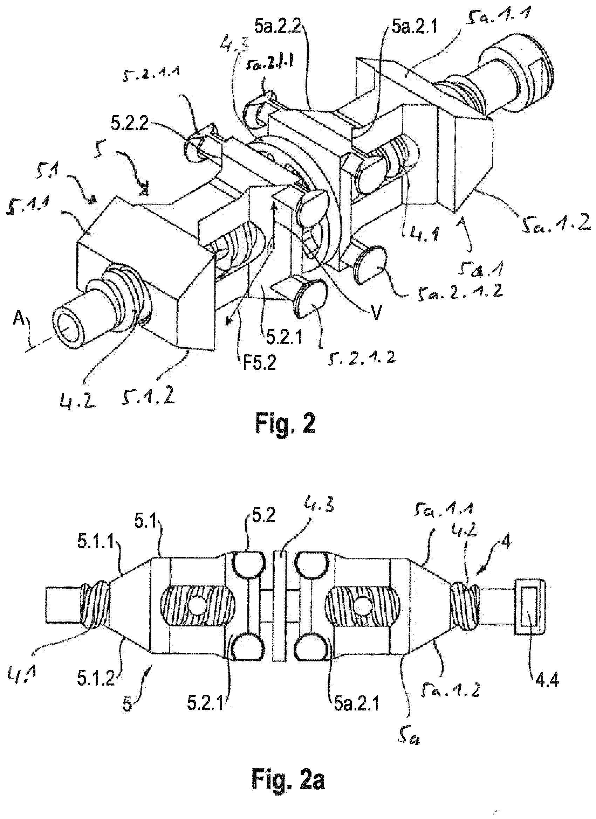

Jeder Doppelkeil 5, 5a weist einen ersten und einen zweiten Rampenkörper 5.1, 5a.1, 5.2, 5a.2 auf, die jeweils in Richtung der Achse A hintereinander angeordnet und von dem Rad 4.3 fort nach außen gerichtet sind. Die Rampen 5.1.1 und 5.1.2 des Rampenkörpers 5.1 laufen von dem Rad 4 fort nach außen aufeinander zu und zwar folgendermaßen (Fig. 2): Der Rampenkörper 5.1 weist als obere und untere Rampenfläche jeweils eine Rampe 5.1.1 und 5.1.2 auf, die jeweils eine Flächennormale F5 mit einem endlichen Winkel ungleich 90° zu einer Vertikalen V zur Achse A aufweisen (Fig. 2, 2a). Für die anderen Rampen der Rampenkörper gilt entsprechendes.

Der Kontaktkörper 2.1 (und ebenso der Kontaktkörper 2.2) weist zur Rampe 5.1.1 eine Gegenfläche 2.6 mit etwa oder genau gleicher Neigung wie die Rampen 5.1.1, 5.1.2 auf, gegen die die Rampe 5.1.1 bzw. 5.1.2 wirkt, wenn unter Verdrehen des Gewindekörpers 4 der Doppelkeil 5 und der Rampenkörper 5.1 und damit die Rampen 5.1.1, 5.1.2 von dem Rad 4.3 fort entlang der Achse A nach außen (in der Abbildung nach links) bewegt wird.

Für den Rampenkörper 5a.1 und die an diesem befindlichen Rampen 5a.1.1, 5a.1.2 gilt grundsätzlich das Gleiche, wobei auf der Innenseite des Kontaktkörpers 2.1 die entsprechende Gegenfläche 2.1.3 ausgebildet ist. Entsprechendes gilt hinsichtlich der korrespondierenden Rampen des Kontaktkörpers 2.2 und für die untere Rampe 5a.1.2 und entsprechende Gegenflächen der unteren Kontaktkörper 2.3 und 2.4.

Der Doppelkeil 5 weist zum Rampenkörper 5.1 in Achsrichtung A nach innen versetzt, wie gesagt, den Rampenkörper 5.2 mit Rampen 5.2.1 und 5.2.2 auf, die vertikal ausgerichtet sind (auch Fig. 3), ebenfalls von dem Rad 4.3 nach außen hin schräg aufeinander zu laufen und jeweils eine Flächennormale F5.2 aufweisen, die horizontal gerichtet ist, d.h. senkrecht zur Vertikalen V, und mit der Achse A eine horizontale Ebene aufspannt (Fig. 3).

Die Rampe 5.2.1 wirkt mit einer ebenfalls vertikal ausgerichteten schräg verlaufenden Gegenfläche 2.4.2 am Kontaktkörper 2.4 und ebenso mit einer entsprechenden Gegenfläche am vorderen oberen Kontaktkörper 2.3 zusammen und kann, wenn der Doppelkeil 5 durch Drehen des Gewindekörpers 4 von dem Rad 4.3 nach außen fortbewegt wird, derart den Kontaktkörper 2.4 (und auch den Kontaktkörper 2.3) nach außen bewegen, bis die Rampe 5.2.1 außer Eingriff mit der Gegenfläche 2.4.2 (und der entsprechenden Gegenfläche des Kontaktkörpers 2.3) gerät, so dass anschließend bei weiterer Schraubbewegung des Gewindekörpers 4 der Doppelkeil 5 sich entlang des Kontaktkörpers 2.4 und auch des Kontaktkörpers 2.3 bewegen kann.

Entsprechendes gilt für die Rampe 5.2.2 und die entsprechende Gegenfläche an den Kontaktkörpern 2.1 und 2.2 sowie die korrespondierenden Rampen 5a.2.1, 5a.2.2 des Rampenkörpers 5a.2 am Doppelkeil 5a (Fig. 3, 3a), wobei insofern auf die vorstehende Erläuterung hinsichtlich des Zusammenwirkens der Rampe 5.2.1 und der Gegenfläche 2.4.2 verwiesen wird.

In der ersten Ausgestaltung der Fig. 1-7a weist der Rampenkörper 5.2 sich seitlich von ihm fort und über die Rampen 5.2.1 hinaus erstreckende obere und untere Ansätze 5.2.1.2 auf (Fig. 2), so der letztere in einen Schlitz 2.1.4 des Kontaktkörpers 2.1 eingreift und derart diesen bei der lateralen Auseinanderbewegung der Kontaktkörper aufgrund der Kontaktflächen der Rampenkörper 5.2 relativ zueinander horizontal führt. Entsprechendes gilt für die zweiten Ansätze der Rampenkörper 5.2 und 5a.2 und zugeordneten Schlitze 2.3.4 der Kontaktkörper 2.1-2.4.

Der Schlitz 2.1.4 weist in seinem zu den Stirnseiten gerichteten Bereich Abkantungen zueinander auf. Hierdurch wird eine Führung gewährleistet, wenn sich die Kontaktkörper 2.1 und 2.3 bzw. auch 2.2 und 2.4 unter Einwirkung der Rampenkörper 5.1, 5a.1 in vertikaler Richtung auseinanderbewegen, wie dies oben beschrieben wurde.

Entsprechendes gilt für die aus den Zeichnungen ersichtlichen Ansätze im Bereich des Rampenkörpers 5a.2 und zugeordnete Schlitze wie beispielsweise 2.3.4a., wozu diesbezüglich sowie zu entsprechenden Ansätzen auf der Gegenseite der beiden genannten Rampenkörper und zu entsprechenden Schlitzen in den Kontaktkörpern 2.1 und 2.2 auf die vorstehende Beschreibung verwiesen wird.

Schließlich sind beispielsweise zwischen den Kontaktkörpern 2.1 und 2.2 noch Führungsstäbe 6.1, 6.2 ausgebildet, die in den beiden Kontaktkörpern 2.1, 2.2 verschieblich sind und die beiden Kontaktkörper bei ihrer lateralen Auseinanderbewegung und vertikalen Hubbewegung relativ zueinander führen.

Entsprechende Führungsstäbe sind in den unteren Kontaktkörpern 2.3 und 2.4 vorgesehen, die diese Körper bei der genannten lateralen und einer Absenkbewegung ebenfalls zueinander führen.

Den Figuren, insbesondere der Fig. 1 ist zu entnehmen, dass der axiale Abstand vom Rampenkörper 5.2 und damit dessen Rampe 5.2.1 zum Rampenkörper 5.1 bzw. dessen Rampe 5.1.1 (und den entsprechenden nicht sichtbaren Rampen) geringer ist als der axiale Abstand zwischen der Gegenfläche 2.4.2 und der Gegenfläche 2.6 bzw. der entsprechenden Gegenfläche des jeweils gleichen Kontaktkörpers. Entsprechendes gilt für den Abstand der Rampenkörper 5a.2 und 5a.1 bzw. deren Rampen im Verhältnis zu den entsprechenden Gegenflächen an den Kontaktkörpern.

Damit wird erreicht, dass sowohl die oberen Kontaktkörper 2.1 und 2.2 als auch die unteren Kontaktkörper 2.3 und 2.4 zunächst in einem ersten Schritt durch die Rampenkörper 5.2 und 5a.2 bzw. deren Rampen lateral seitlich auseinandergefahren werden und erst anschließend, also zeitlich versetzt zu dem vorgenannten Schritt, die Kontaktkörper 2.1 und 2.3 sowie die Kontaktkörper 2.2 und 2.4 durch die Rampenkörper 5.1 und 5a.1 bzw. den Rampen in senkrechter bzw. vertikaler Richtung auseinandergefahren werden und damit erst im lateral verbreiterten Zustand die Kontaktkörper 2.1-2.4 gegen den oberen und unteren Wirbelkörper verfahren werden, wodurch die Gefahr einer Beschädigung derselben reduziert wird.

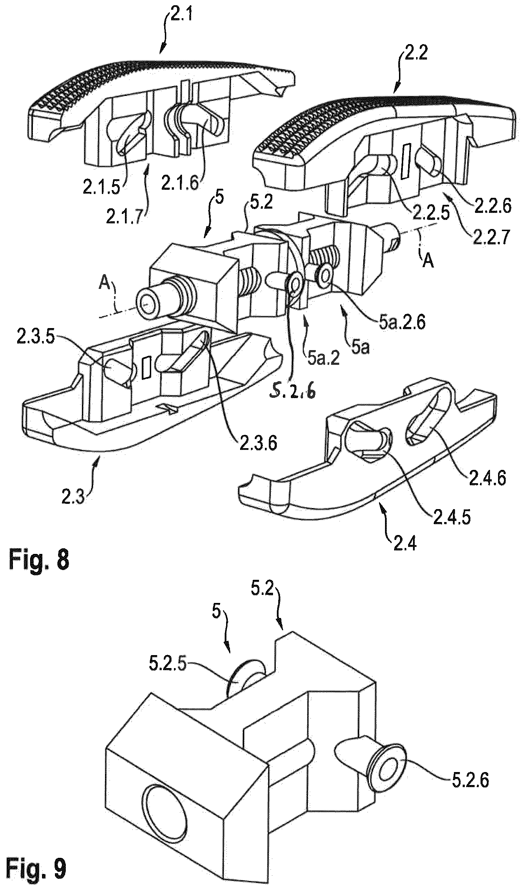

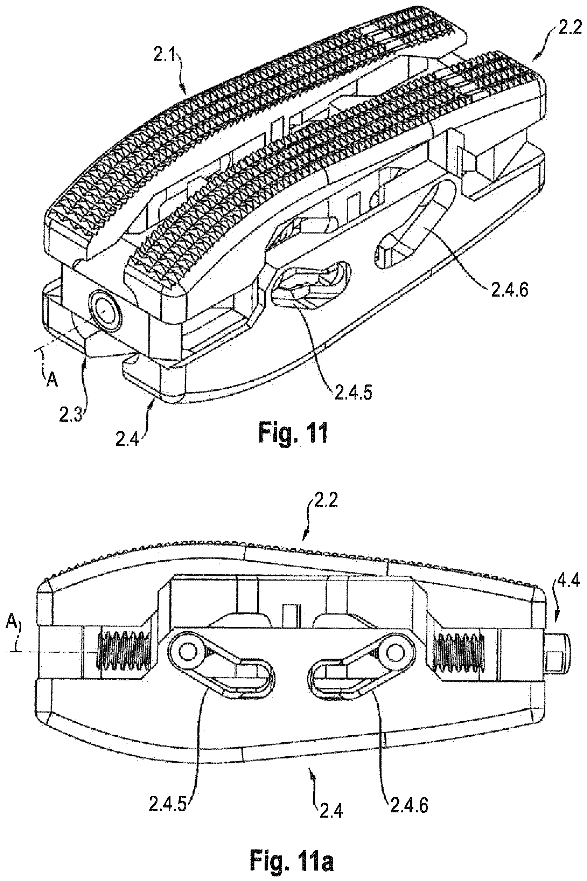

Die Ausführungsform des erfindungsgemäßen Zwischenwirbelimplantats ist in den Fig. 8 bis 11a dargestellt. Diese Ausführungsform weist grundsätzlich und weitgehend die gleiche Ausgestaltung wie die erste Ausführungsform der Fig. 1 bis 7a auf. Insofern werden gleiche Teile mit gleichen Bezugszeichen versehen und es wird zur Beschreibung auf die vorstehende Beschreibung der ersten Ausführungsform verwiesen. Der wesentliche Unterschied aus der etwas anderen Ausgestaltung der Rampenkörper 5.2, 5.2a ist der Doppelkeil 5, 5a. Der Unterschied liegt darin, dass diese statt der zweiseitlichen Ansätze der entsprechenden Rampenkörper der Ausgestaltung der Fig. 1 bis 7a auf jeder Seite einen seitlichen Ansatz 5.2.5 und 5.2.6 des Rampenkörpers 5.2 und 5a.2.6 des Rampenkörpers 5a.2 zeigen (der auf der anderen Seite am Rampenkörper 5a.2 vorhandene Ansatz ist in den Darstellungen nicht ersichtlich). Die Ansätze 5.2.5, 5.2.6 und 5a.2.6 sind auf mittlerer Höhe des Rampenkörpers 5.2 bzw. 5a.2 angeordnet. Sie greifen in zunächst horizontal, dann schräg verlaufende Schlitze 2.1.5, 2.1.6, 2.2.5, 2.2.6, 2.3.5, 2.3.6, 2.4.5, 2.4.6 ein, wobei der äußere geneigte Abschnitt der Schlitze 2.1.5, 2.1.6, 2.2.5, 2.2.6 der oberen Kontaktkörper 2.1, 2.2 nach außen hin abwärts verläuft, während die äußeren geneigten Abschnitte der Schlitze 2.3.5, 2.3.6, 2.4.5, 2.4.6 der unteren Kontaktkörper 2.3, 2.4 nach außen hin aufwärts verlaufen.

Dies bewirkt, dass wenn die Ansätze 5.2.5 und 5.2.6 (sowie die entsprechenden Ansätze des Rampenkörpers 5a) in die geneigten Abschnitte der genannten Schlitze eintreten die unteren Kontaktkörper 2.3 und 2.4 nach unten und die oberen Kontaktkörper 2.1 und 2.2 nach oben gedrückt werden und damit eine vertikale Spreizung bewirkt wird. Die vorangehende horizontale Spreizung der Kontaktkörper 2.1 und 2.2 relativ zueinander und 2.3 sowie 2.4 relativ zueinander erfolgt in der gleichen Weise, wie unter Bezug auf die erste Ausgestaltung beschrieben.

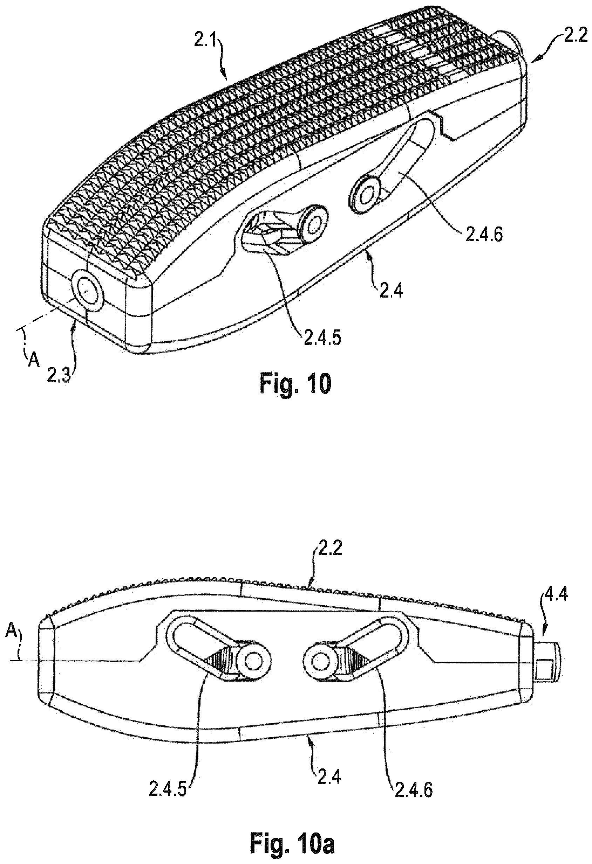

Damit die Ansätze 5.2.5 und 5.2.6 sowie auch 5a.2.6 (und der entsprechende gegenüberliegende Ansatz des Rampenkörpers 5a.2) jeweils in einen Schlitz eines unteren Kontaktkörpers und eines oberen Kontaktkörpers gleichzeitig eingreifen können, um die entsprechenden Vertikalspreizungen zu bewegen, also beispielsweise der Ansatz 5.2.5 sowohl in den Schlitz 2.3.5 des Kontaktkörpers 2.3 als auch in den Schlitz 2.1.5 des Kontaktkörpers 2.1 müssen die Schlitze sich in der komprimierten Grundkonfiguration (Fig. 10, 10a) überlappen. Demgemäß greift ein die Schlitze 2.1.5, 2.1.6 bzw. 2.2.5, 2.2.6 aufweisender horizontaler Bereich 2.1.7, 2.2.7 in vertikaler Richtung innerhalb einer Seitenwandung des entsprechenden unteren Kontaktkörpers 2.3 bzw. 2.4 im Inneren ein, wodurch es zur Überlagerung der Schlitze in ihrem horizontalen Bereich kommt, wie bei gedanklicher Zusammenführung der Kontaktkörper 2.1 und 2.3 einerseits und 2.2 und 2.4 in der Fig. 8 andererseits ohne weiteres ersichtlich.

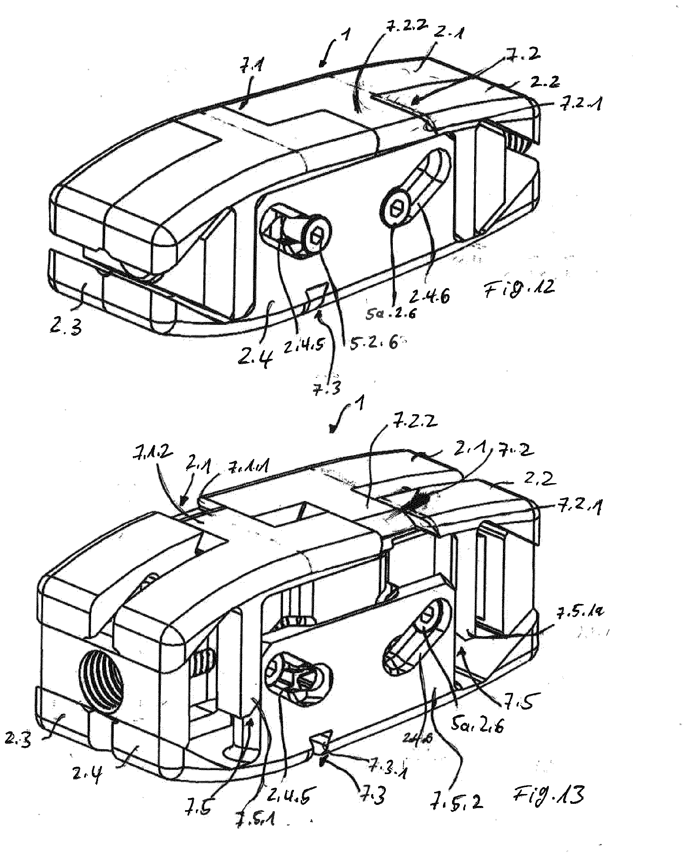

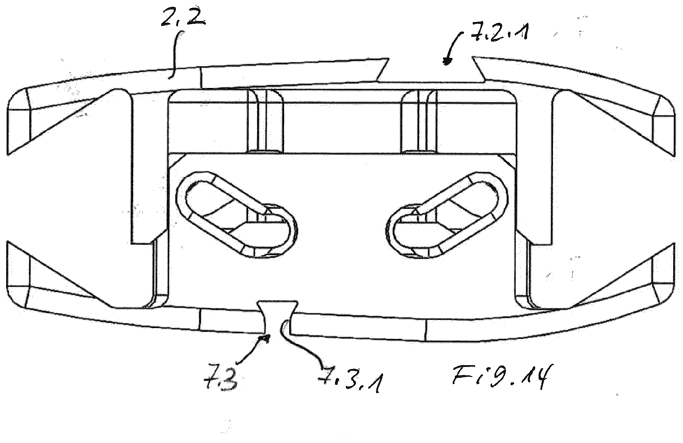

Die Fig. 12 bis 16 zeigen eine weitere Abwandlung des Zwischenwirbelimplantats der vorangehenden Figuren. Gleiche Teile sind mit gleichen Bezugszeichen bezeichnet. Das Zwischenwirbelimplantat der Fig. 12 bis 16 weist ebenfalls obere Kontaktkörper 2.1, 2.2 und untere Kontaktkörper 2.3, 2.4 auf. In der Fig. 12 ist das Zwischenwirbelimplantat 1 im komprimierten Zustande dargestellt, in der Fig. 13 in einem - lediglich - seitlich oder horizontal expandierten Zustand.

Das Implantat 1 weist zunächst Linearführungen 7.1, 7.2 der beiden oberen Kontaktkörper 2.1, 2.2 auf (Fig. 12, 13). Weiter weist es Linearführungen 7.3, 7.4 der beiden unteren Kontaktkörper 2.3, 2.4 auf (Fig. 12, 13, 14, 16). Die Linearführungen 7.1-7.4 dienen zur seitlichen oder horizontalen Führung jeweils der oberen Kontaktkörper 2.1, 2.2 bzw. der unteren Kontaktkörper 2.3, 2.4 zueinander. Weiter sind entsprechende - vertikale - Linearführungen der unmittelbar übereinanderliegenden Kontaktkörper 2.1 und 2.3 bzw. 2.2 und 2.4 vorgesehen, wobei in den Abbildungen, genauer in der Fig. 13, lediglich eine vertikale Linearführung 7.5 der Kontaktkörper 2.2, 2.4 dargestellt ist, die weiter unten erläutert wird.

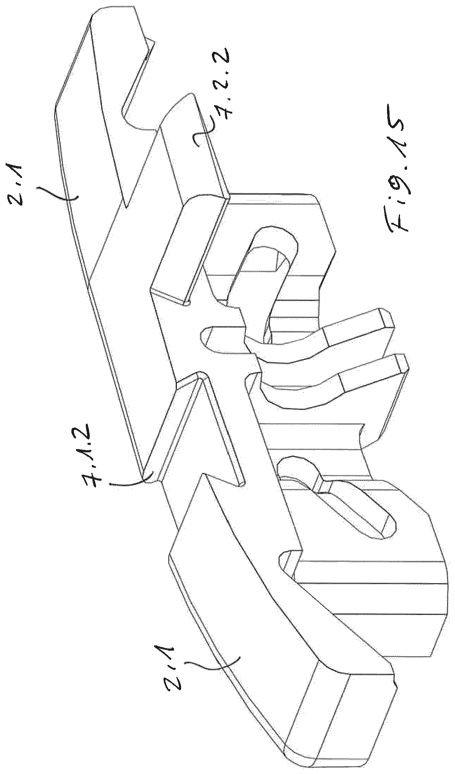

Jede Linearführung 7.1-7.2 besteht aus einer Nut-Feder-Führung, insbesondere einer Schwalbenschwanzführung mit Hinterschneidungen, wie dies letztere insbesondere bei der Führung 7.3 in den Fig. 12, 13, 16, den Führungen 7.1 und 7.2 der Fig. 13 und auch der Fig. 15 dargestellt und ersichtlich ist. Entsprechendes gilt auch für die Führung 7.5 der Fig. 13.

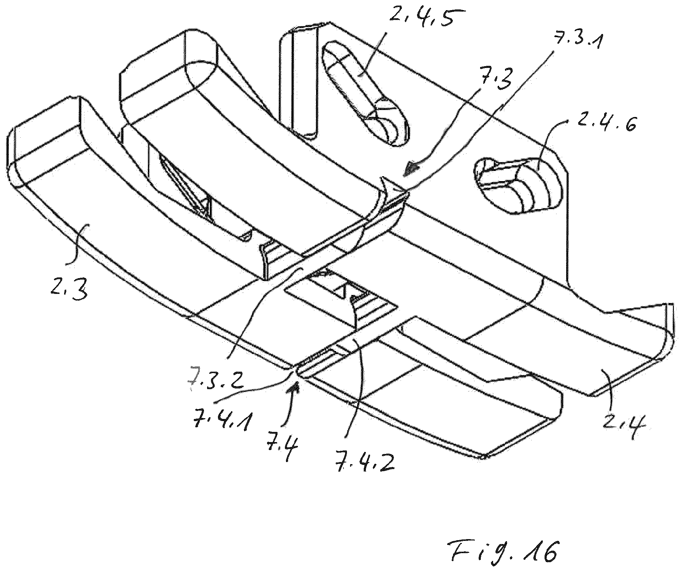

Jede Linearführung weist demgemäß eine Nut und einen in diesen eingreifenden, üblicherweise als "Feder" bezeichneten Vorsprung oder Ansatz auf. Bei der Linearführung 7.1 sind dies die Nut 7.1.1 und die Feder 7.1.2, bei der Linearführung 7.2 die Nut 7.2.1 und die Feder oder der Vorsprung 7.2.2 (jeweils insbesondere Fig. 13). Bei der Linearführung 7.3 sind dies die Nut 7.3.1 und die Feder 7.3.2 und bei der Linearführung 7.4 die Nut 7.4.1 und die Feder 7.4.2 (jeweils Fig. 16).

Bei der - vertikalen - Linearführung 7.5, die in der Fig. 13 dargestellt ist, ist die Nut zwischen den beiden nach unten führenden Führungsvorsprüngen 7.5.1 und 7.5.1a gebildet und die entsprechende Feder oder der Vorsprung der Führung 7.5 wird durch die Seitenwandung 7.5.2 des - von der Angriffsseite aus - rechten unteren Tragkörpers gebildet. Dabei sind die miteinander zusammenwirkenden Seitenwandungen des Vorsprungs oder der Feder 7.5.2 und der Führungsvorsprünge 7.5.1, 7.5.1a ebenfalls schwalbenschwanzförmig mit Hinterschneidungen ausgebildet, wie dies in der Fig. 13 ersichtlich ist.

Eine vertikale Linearführung der übereinanderliegenden Tragköper 2.1, 2.3 ist in gleicher Weise ausgebildet wie die Linearführung 7.5.

Durch die Linearführungen wird erreicht, dass auftretende Querkräfte durch diese aufgenommen werden und insbesondere die Doppelschraube 4 und mit diesen zusammenwirkenden Gewinden der Keilkörper von solchen Kräften befreit oder zumindest entlastet werden.

Das erfindungsgemäße Zwischenwirbelkörperimplantat 1 wird in allen Ausführungsformen grundsätzlich in folgender Weise eingeführt und platziert:
Zunächst wird ein Zugang zum Zwischenwirbelkörperfach gelegt, wie dies beispielsweise in der WO 2014/146797 beschrieben ist.

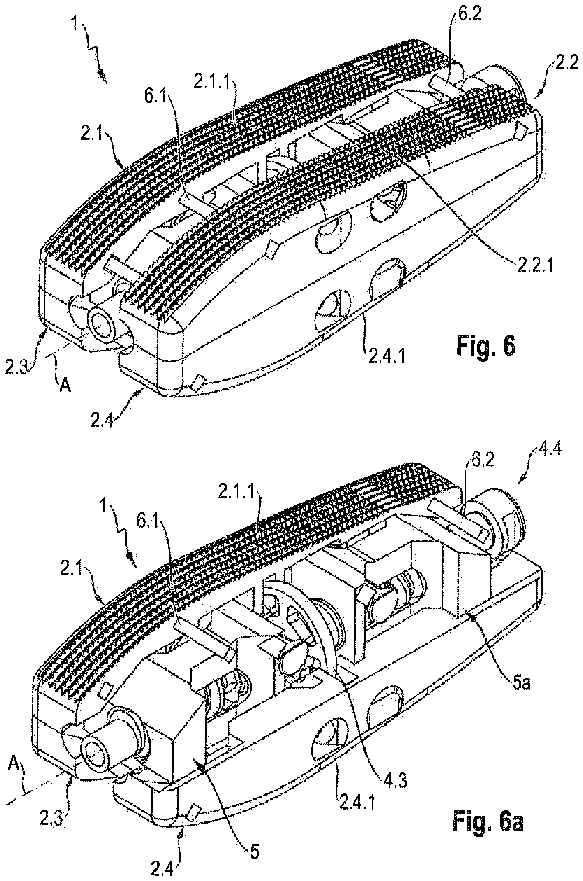

Durch die Zugangshülse wird dann das Zwischenwirbelimplantat 1 in seinem komprimierten Zustand entsprechend der Ausgestaltung der Fig. 5 eingeführt. Damit ist dann die Zwischenkonfiguration der Fig. 6, 6a erreicht. Wenn das Zwischenwirbelimplantat 1 seine Position zwischen den beiden Wirbeln, einem unter ihm befindlichen und einem über ihm befindlichen Wirbel erreicht hat, wird durch die Einführhülse mittels eines Werkzeugs an einem Betätigungsteil am proximalen Ende des Zwischenwirbelimplantats 1 angegriffen und über das Werkzeug der Gewindekörper 4 verdreht. Da die beiden Doppelkeile 5, 5a sich nicht mitdrehen können, werden sie durch die Gewindeverbindung zwischen ihnen und den entgegengerichteten Außengewinden des Gewindekörpers aus der Ausgangsposition in entgegengesetzter Richtung voneinander fortbewegt, wie dies im Übergang von der Fig. 2 zur Fig. 3 ersichtlich ist.

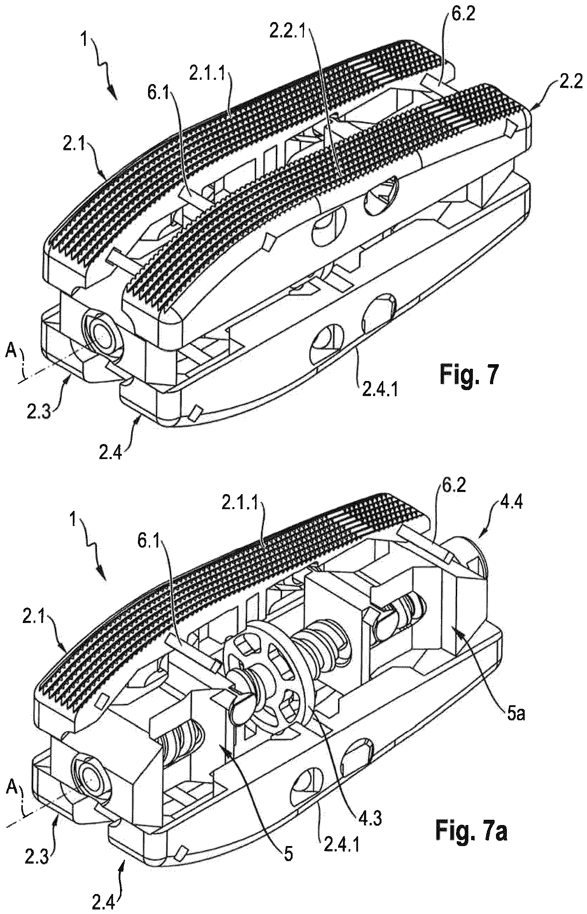

Dabei greifen zunächst die Rampenkörper 5.2 und 5a.2 an den entsprechenden Gegenflächen der Kontaktkörper 2.1-2.4 an und verfahren diese lateral auseinander, bis die Gegenfläche der Kontaktkörper - beispielsweise 2.4.2 - die entsprechende Rampe freigibt, so dass sie beim weiteren Verdrehen des Gewindekörpers 4 sich entlang des Tragkörpers bewegen kann. Gleichzeitig - d.h. nach vollständigem lateralen Auseinanderfahren der Tragkörper - greift der Rampenkörper 5.1 mit seinen Rampen an den entsprechenden Gegenflächen - wie 2.6 - der Kontaktkörper an, so dass beim weiteren Verdrehen des Gewindekörpers diese durch die beiden genannten Flächen angehoben werden, bis die Konfiguration der Fig. 7, 7a erreicht ist.

Bei diesem letzten Verfahrensschritt werden die Kontaktkörper mit ihren äußeren Seitenflächen gegen die Wirbelkörper verfahren und damit zumindest eine Verspannung der Teile Wirbelkörper und Zwischenwirbelimplantat erreicht.

Dadurch, dass die Kontaktkörper erst im lateral gespreizten Zustand gegen die Wirbelkörper verfahren, wird die Gefahr einer Beschädigung derselben wesentlich reduziert bzw. ausgeschlossen.

## Patentansprüche

1. Zwischenwirbelimplantat
- mit mindestens zwei oberen und zwei unteren Kontaktkörpern (2.1-2.4) mit Kontaktflächen (2.1.1-2.4.1),
- mit einem Aktuator (3) mit einem eine Erstreckungsachse (A) aufweisenden Gewindekörper (4), der mit hintereinander angeordneten gegenläufigen Gewinden (4.1, 4.2) versehen ist,
- mit auf dem Gewindekörper (4) axial beweglich aufsitzenden, durch Verdrehung desselben entlang diesem verfahrbaren Keilen mit wenigstens an Gegenflächen zumindest eines Teils der Kontaktkörper angreifenden, unter einem endlichen Winkel kleiner 90°aufeinander zu verlaufenden Rampen (5.2.1, 5a.2.1) mindestens eines Rampenkörpers (5.2, 5a.2) eines Keils,
**dadurch gekennzeichnet,**
- **dass** die Keile als Doppelkeile mit in Richtung der Erstreckungsachse (A) zwei hintereinander angeordnete Rampenkörper ausgebildet sind,
- **dass** die Rampen (5.1.1, 5.1.2; 5a.1.1, 5a.1.2) des einen Rampenkörpers (5.1, 5.2,) zu den Rampen (5.2.1, 5.2.2, 5a.2.1, 5a.2.2) des anderen Rampenkörpers (5a.1, 5a.2) unterschiedliche Ausrichtung aufweisen.
- **dass** die Rampen (5.2.1, 5.2.2; 5a.2.1, 5a.2.2) des ersten Rampenkörpers (5.2, 5a.2) seitlich direkt an den Kontaktkörpern (2.1-2.4) angreifen, während die Rampen (5.1.1, 5.1.2; 5a.1.1, 5a.1.2) des zweiten Rampenkörpers (5.1, 5a.1) in vertikaler Richtung direkt an den Kontaktkörpern (2.1-2.4) angreifen.

2. Zwischenwirbelimplantat nach Anspruch 1, **dadurch gekennzeichnet, dass** die Rampen (5.2.1, 5a.2.1) von ersten Rampenkörpern (5.2, 5a.2) vertikal mit horizontaler Flächennormale (H) ausgerichtet sind und die Flächennormalen (F) der Rampen (5.1.1, 5a.1.1) von zweiten Rampenkörpern (5.1, 5a.1) in hierzu unterschiedlicher Ausrichtung einen endlichen Winkel ungleich 90° zur Vertikalen (V) einschließen.

3. Zwischenwirbelimplantat nach Anspruch 1 oder 2, **dadurch gekennzeichnet, dass** die Rampen (5.1.1, 5.2.1 bzw. 5a.1.1, 5a.2.1) unterschiedlicher Ausrichtung an unterschiedlichen Rampenkörpern (5.1, 5.2 bzw. 5a.1, 5a.2) angeordnet sind.

4. Zwischenwirbelimplantat nach einem der Ansprüche 1 bis 3, **dadurch gekennzeichnet, dass** mit Rampen (5.1.1, 5.2.1; 5a.1.1, 5a.2.1) unterschiedlicher Ausrichtung unterschiedliche Ausrichtung zusammenwirkende Gegenflächen (2.4.2, 2.6) der Kontaktkörper (2.1-2.4) mit einem gegenüber dem Abstand der Rampenkörper (5.1, 5.2, 5a.1, 5a.2) unterschiedlicher Ausrichtung unterschiedlichen Abstand in Erstreckungsrichtung der Achse (A) des Gewindekörpers (4) des Aktuators aufweisen.

5. Zwischenwirbelimplantat nach einem der vorangehenden Ansprüche, **dadurch gekennzeichnet, dass** der Abstand von Rampen (5.1.1, 5.2.1; 5a.1.1, 5a.2.2) unterschiedlicher Ausrichtung relativ zum Abstand der Gegenflächen (2.4.2, 2.6) an den Kontaktkörpern (2.1-2.4) derart ist, dass bei einer Drehung des Gewindekörpers (4) die Kontaktkörper (2.1-2.4) zumindest lateral auseinanderbewegt und erst anschließend relativ zueinander angehoben werden.

6. Zwischenwirbelimplantat nach einem vorangehenden Ansprüche, **dadurch gekennzeichnet, dass** der Abstand der Rampen (5.1.1, 5.2.1; 5a.1.1, 5a.2.1) relativ zum Abstand der Gegenflächen (2.4.2, 2.6) derart ist, dass die Kontaktkörper (2.1-2.4) zuerst durch die Rampen (5.2.1, 5a.2.1) des ersten Rampenkörpers (5.2, 5a.2) seitlich an den Gegenflächen (2.4.2) der Kontaktkörper (2.1-2.4) angreifen, um diese lateral auseinander zu bewegen und erst bei weiterer Verdrehung die Rampen (5.1.1, 5a.1.1) des zweiten Rampenkörpers (5.1, 5a.1) an den Gegenflächen (2.6) der Kontaktkörper (2.1-2.4) angreifen, um diese anzuheben.

7. Zwischenwirbelimplantat nach einem der vorangehenden Ansprüche, **dadurch gekennzeichnet, dass** der Abstand der Rampen unterschiedlicher Ausrichtung (5.1.1, 5.2.1; 5a.1.1, 5a.2.1) eines Doppelkeils (5 bzw. 5a) geringer ist als der Abstand der zugehörigen Gegenflächen (2.4.1, 2.6).

8. Zwischenwirbelimplantat nach einem der vorangehenden Ansprüche, **dadurch gekennzeichnet, dass** der Aktuator eine fest mit dem Gewindekörper verbundene radiale Scheibe oder ein radiales Rad (4.3) aufweist, das in radial zur Achse (A) des Gewindekörpers (4) gerichtete Schlitze (2.5) der Kontaktkörper (2.1-2.4) zur achssenkrechten Führung derselben eingreift.

9. Zwischenwirbelimplantat nach einem der vorangehenden Ansprüche, **gekennzeichnet durch** zumindest in den oberen Kontaktkörpern (2.1, 2.2) gleitend eingreifende Führungsstäbe zur Führung der Kontaktkörper (2.1, 2.2) relativ zueinander.

10. Zwischenwirbelimplantat nach einem der vorangehenden Ansprüche, **dadurch gekennzeichnet, dass** die äußere Kontaktfläche von oberen Kontaktkörpern (2.1, 2.2) zu der äußeren Kontaktfläche der unteren Kontaktkörper (2.3, 2.4) einen Winkel zwischen 5° und 15°, vorzugsweise zwischen 9° und 11° einschließen.

11. Zwischenwirbelimplantat, **dadurch gekennzeichnet, dass** die Rampenkörper (5.1, 5.2, 5a.1, 5a.2) an einem Doppelkeil einstückig mit diesem ausgebildet sind.

12. Zwischenwirbelimplantat nach einem der vorangehenden Ansprüche, **dadurch gekennzeichnet, dass** übereinanderliegende und/oder nebeneinanderliegende Kontaktkörper (2.1, 2.3; 2.2, 2.4 bzw. 2.1, 2.2; 2.3, 2.4) über Linearführungen beweglich zueinander verbunden sind.

13. Zwischenwirbelimplantat nach Anspruch 12, **dadurch gekennzeichnet, dass** zumindest eine Linearführung eine Nut-Feder-Führung, vorzugsweise eine Schwalbenschwanzführung ist.

## Claims

1. Intervertebral implant
- comprising at least two upper and two lower contact bodies (2.1-2.4) having contact surfaces (2.1.1-2.4.1),
- comprising an actuator (3) having a threaded body (4) which has an extension axis (A) and is provided with opposite-handed threads (4.1, 4.2) arranged one behind the other,
- comprising wedges which sit on the threaded body (4) in an axially moveable manner, can be moved along said threaded body by rotating same, and comprise ramps (5.2.1, 5a.2.1) of at least one ramp body (5.2, 5a.2) of a wedge, which ramps engage at least with counter-surfaces of at least some of the contact bodies and extend toward one another at a finite angle of less than 90°,
**characterized in that**
- the wedges are designed as double wedges having, in the direction of the extension axis (A), two ramp bodies arranged one behind the other,
- the ramps (5.1.1, 5.1.2; 5a.1.1, 5a.1.2) of one ramp body (5.1, 5.2) are oriented differently to the ramps (5.2.1, 5.2.2, 5a.2.1, 5a.2.2) of the other ramp body (5a.1, 5a.2),
- the ramps (5.2.1, 5.2.2; 5a.2.1, 5a.2.2) of the first ramp body (5.2, 5a.2) engage directly with the contact bodies (2.1-2.4) laterally, while the ramps (5.1.1, 5.1.2; 5a.1.1, 5a.1.2) of the second ramp body (5.1, 5a.1) engage directly with the contact bodies (2.1-2.4) in the vertical direction.

2. Intervertebral implant, **characterized in that** the ramps (5.2.1, 5a.2.1) of the first ramp bodies (5.2, 5a.2) are oriented vertically with a horizontal surface normal (H), and the surface normals (F) of the ramps (5.1.1, 5a.1.1) of the second ramp bodies (5.1, 5a.1), which have a different orientation, include a finite angle other than 90° to the vertical (V).

3. Intervertebral implant according to either claim 1 or claim 2, **characterized in that** the ramps (5.1.1, 5.2.1 or 5a.1.1, 5a.2.1) of different orientations are arranged on different ramp bodies (5.1, 5.2 or 5a.1, 5a.2).

4. Intervertebral implant according to any of claims 1 to 3, **characterized in that** counter-surfaces (2.4.1, 2.6) of the contact bodies (2.1- 2.4) that interact with differently oriented ramps (5.1.1, 5.2.1; 5a.1.1, 5a.2.1) and have a different orientation in relation to the spacing of the ramp bodies (5.1, 5.2, 5a.1, 5a.2) have a different spacing in the extension direction of the axis (A) of the threaded body (4) of the actuator.

5. Intervertebral implant according to any of the preceding claims, **characterized in that** the spacing of the differently oriented ramps (5.1.1, 5.2.1; 5a.1.1, 5a.2.2) relative to the spacing of the counter-surfaces (2.4.2, 2.6) on the contact bodies (2.1-2.4) is such that, when the threaded body (4) rotates, the contact bodies (2.1-2.4) are moved apart from one another at least laterally and are only then raised relative to one another.

6. Intervertebral implant according to any of the preceding claims, **characterized in that** the spacing of the ramps (5.1.1, 5.2.1; 5a.1.1, 5a.2.1) relative to the spacing of the counter-surfaces (2.4.2, 2.6) is such that the contact bodies (2.1-2.4) first engage laterally with the counter-surfaces (2.4.2) of the contact bodies (2.1-2.4) by means of the ramps (5.2.1, 5a.2.1) of the first ramp body (5.2, 5a.2) in order to move them laterally apart from one another, and only upon further rotation do the ramps (5.1.1, 5a.1.1) of the second ramp body (5.1, 5a.1) engage with the counter-surfaces (2.6) of the contact bodies (2.1-2.4) in order to raise them.

7. Intervertebral implant according to any of the preceding claims, **characterized in that** the spacing between the differently oriented ramps (5.1.1, 5.2.1; 5a.1.1, 5a.2.1) of a double wedge (5 or 5a) is less than the spacing between the associated counter-surfaces (2.4.1, 2.6).

8. Intervertebral implant according to any of the preceding claims, **characterized in that** the actuator has a radial disk rigidly connected to the threaded body or has a radial wheel (4.3) which engages in slots (2.5) of the contact bodies (2.1-2.4) that are oriented radially to the axis (A) of the threaded body (4) in order to guide said contact bodies perpendicularly to the axis.

9. Intervertebral implant according to any of the preceding claims, **characterized by** guide rods which slidably engage at least in the upper contact bodies (2.1, 2.2) and are intended for guiding the contact bodies (2.1, 2.2) relative to one another.

10. Intervertebral implant according to any of the preceding claims, **characterized in that** the outer contact surface of the upper contact bodies (2.1, 2.2) and the outer contact surface of the lower contact bodies (2.3, 2.4) include an angle of between 5° and 15°, preferably between 9° and 11°.

11. Intervertebral implant, **characterized in that** the ramp bodies (5.1, 5.2, 5a.1, 5a.2) on a double wedge are formed integrally therewith.

12. Intervertebral implant according to any of the preceding claims, **characterized in that** contact bodies (2.1, 2.3; 2.2, 2.4 or 2.1, 2.2; 2.3, 2.4) above and/or next to one another are movably connected to one another via linear guides.

13. Intervertebral implant according to claim 12, **characterized in that** at least one linear guide is a tongue-and-groove guide, preferably a dovetail guide.

## Revendications

1. Implant intervertébral
- comportant au moins deux corps de contact supérieurs et deux corps de contact inférieurs (2.1-2.4) comportant des surfaces de contact (2.1.1-2.4.1),
- comportant un actionneur (3) comportant un corps fileté (4) présentant un axe d'extension (A), lequel corps fileté est pourvu de filetages (4.1, 4.2) en sens contraire et disposés les uns derrière les autres,
- comportant des cales posées de manière mobile axialement sur le corps fileté (4), déplaçables le long du corps fileté par rotation de celui-ci, les cales comportant des rampes (5.2.1, 5a.2.1) d'au moins un corps de rampe (5.2, 5a.2) d'une cale, lesquelles rampes viennent en prise au moins avec des contre-surfaces d'au moins une partie des corps de contact et s'étendent les unes vers les autres selon un angle fini inférieur à 90°,
**caractérisé en ce**
- **que** les cales sont conçues sous forme de cales doubles comportant, dans la direction de l'axe d'extension (A), deux corps de rampe disposés l'un derrière l'autre,
- **en ce que** les rampes (5.1.1, 5.1.2 ; 5a.1.1, 5a.1.2) d'un corps de rampe (5.1, 5.2,) présentent une orientation différente par rapport aux rampes (5.2.1, 5.2.2, 5a.2.1, 5a.2.2) de l'autre corps de rampe (5a.1, 5a.2).
- **en ce que** les rampes (5.2.1, 5.2.2 ; 5a.2.1, 5a.2.2) du premier corps de rampe (5.2, 5a.2) viennent en prise latéralement directement avec les corps de contact (2.1-2.4), tandis que les rampes (5.1.1, 5.1.2 ; 5a.1.1, 5a.1.2) du second corps de rampe (5.1, 5a.1) viennent en prise, en direction verticale, directement avec les corps de contact (2.1-2.4).

2. Implant intervertébral selon la revendication 1, **caractérisé en ce que** les rampes (5.2.1, 5a.2.1) de premiers corps de rampe (5.2, 5a.2) sont orientées verticalement avec une normale de surface horizontale (H), et les normales de surface (F) des rampes (5.1.1, 5a.1.1) de seconds corps de rampe (5.1, 5a.1) forment, dans une orientation différente, un angle fini différent de 90° par rapport à la verticale (V).

3. Implant intervertébral selon la revendication 1 ou 2,
**caractérisé en ce que** les rampes (5.1.1, 5.2.1 ou 5a.1.1, 5a.2.1) d'orientation différente sont disposées sur différents corps de rampe (5.1, 5.2 ou 5a.1, 5a.2).

4. Implant intervertébral selon l'une des revendications 1 à 3,
**caractérisé en ce que** des contre-surfaces (2.4.2, 2.6) d'orientation différente des corps de contact (2.1-2.4), coopérant avec des rampes (5.1.1, 5.2.1 ; 5a.1.1, 5a.2.1) d'orientation différente, présentent un espacement dans la direction d'extension de l'axe (A) du corps fileté (4) de l'actionneur, qui est différent de l'espacement entre les corps de rampe (5.1, 5.2, 5a.1, 5a.2) d'orientation différente.

5. Implant intervertébral selon l'une des revendications précédentes, **caractérisé en ce que** l'espacement des rampes (5.1.1, 5.2.1 ; 5a.1.1, 5a.2.2) d'orientation différente par rapport à l'espacement des contre-surfaces (2.4.2, 2.6) sur les corps de contact (2.1-2.4) est tel que, lors d'une rotation du corps fileté (4), les corps de contact (2.1-2.4) sont écartés au moins latéralement et ne sont soulevés que par la suite les uns par rapport aux autres.

6. Implant intervertébral selon l'une des revendications précédentes, **caractérisé en ce que** l'espacement des rampes (5.1.1, 5.2.1 ; 5a.1.1, 5a.2.1) par rapport à l'espacement des contre-surfaces (2.4.2, 2.6) est tel que les corps de contact (2.1-2.4) viennent d'abord en prise latéralement avec les contre-surfaces (2.4.2) des corps de contact (2.1-2.4) par les rampes (5.2.1, 5a.2.1) du premier corps de rampe (5.2, 5a.2) afin de les séparer latéralement et ce n'est que lorsque la rotation se poursuit que les rampes (5.1.1, 5a.1.1) du second corps de rampe (5.1, 5a.1) viennent en prise avec les contre-surfaces (2.6) des corps de contact (2.1-2.4) afin de les soulever.

7. Implant intervertébral selon l'une des revendications précédentes, **caractérisé en ce que** l'espacement entre les rampes (5.1.1, 5.2.1 ; 5a.1.1, 5a.2.1) d'orientation différente d'une double cale (5 ou 5a) est inférieur à l'espacement entre les contre-surfaces (2.4.1, 2.6) correspondantes.

8. Implant intervertébral selon l'une des revendications précédentes, **caractérisé en ce que** l'actionneur présente un disque radial ou une roue radiale (4.3) solidaire du corps fileté, lequel disque radial/laquelle roue radiale pénètre dans des fentes (2.5) des corps de contact (2.1-2.4), orientées radialement par rapport à l'axe (A) du corps fileté (4) pour le guidage du disque radial/de la roue radiale perpendiculairement à l'axe.

9. Implant intervertébral selon l'une des revendications précédentes, **caractérisé par** des barres de guidage pénétrant de manière coulissante au moins dans les corps de contact (2.1, 2.2) supérieurs pour le guidage des corps de contact (2.1, 2.2) les uns par rapport aux autres.

10. Implant intervertébral selon l'une des revendications précédentes, **caractérisé en ce que** la surface de contact extérieure de corps de contact (2.1, 2.2) supérieurs forme, avec la surface de contact extérieure des corps de contact (2.3, 2.4) inférieurs, un angle compris entre 5° et 15°, de préférence entre 9° et 11°.

11. Implant intervertébral, **caractérisé en ce que** les corps de rampe (5.1, 5.2, 5a.1, 5a.2) sont réalisés sur une cale double, d'un seul tenant avec celle-ci.

12. Implant intervertébral selon l'une des revendications précédentes, **caractérisé en ce que** des corps de contact (2.1, 2.3 ; 2.2, 2.4 ou 2.1, 2.2 ; 2.3, 2.4) superposés et/ou juxtaposés sont reliés entre eux de manière mobile par des guidages linéaires.

13. Implant intervertébral selon la revendication 12, **caractérisé en ce qu'**au moins un guidage linéaire est un guidage à rainure et languette, de préférence un guidage à queue d'aronde.
